# EUROPEAN PATENT SPECIFICATION

(11) **EP 3 261 673 B1**
(45) Date of publication and mention of the grant of the patent: **06.01.2021**
(21) Application number: 16706528.3
(22) Date of filing: 22.02.2016
(51) Int. Cl.: A61K 41/00, A61N 5/10, A61K 38/16, A61K 47/64

(54) **COMBINED VACCINATION/RADIOTERAPY FOR CANCER TREATMENT**
KOMBINIERTE IMPFUNGS-/STRAHLENTHERAPIE ZUR KREBSBEHANDLUNG
VACCINATION/RADIOTHÉRAPIE COMBINÉES POUR LE TRAITEMENT DU CANCER

(30) Priority: 23.02.2015 WO PCT/EP2015/000507
(43) Date of publication of application: 03.01.2018
(73) Proprietor: Institut Curie, 75005 Paris (FR); Centre National de la Recherche Scientifique (C.N.R.S.), 75794 Paris Cedex 16 (FR); Institut Gustave Roussy, 94805 Villejuif Cédex (FR); Assistance Publique-Hôpitaux de Paris (APHP), 75004 Paris (FR); Institut National de la Santé et de la Recherche Médicale, 75654 Paris 13 (FR); Université René Descartes (Paris V), 75006 Paris (FR)
(72) Inventor: DEUTSCH, Eric, 75004 Paris (FR); LUDGER, Johannes, 92400 Courbevoie (FR); MONDINI, Michèle, 75013 Paris (FR); NIZARD, Mevyn, 94260 Fresnes (FR); PERFETTINI, Jean-Luc, 77100 Meaux (FR); TARTOUR, Eric, 75003 Paris (FR); TRAN, Thi, 75018 Paris (FR)
(74) Representative: Barbot, Willy
(86) International application number: PCT/EP2016/000303
(87) International publication number: WO 2016/134838

(56) References cited:
- EP-A1- 2 740 491
- WO-A2-2012/177624
- HAICHEUR N ET AL: "The B subunit of Shiga toxin fused to a tumor antigen elicits CTL and targets dendritic cells to allow MHC class I-restricted presentation of peptides derived from exogenous antigens", THE JOURNAL OF IMMUNOLOGY, THE AMERICAN ASSOCIATION OF IMMUNOLOGISTS, US, vol. 165, no. 6, 15 September 2000 (2000-09-15), pages 3301-3308, XP002228666, ISSN: 0022-1767 cited in the application
- REBECCA G. POMERANTZ ET AL: "The role of epidermal growth factor receptor in head and neck squamous cell carcinoma", CURRENT ONCOLOGY REPORTS, vol. 5, no. 2, 1 April 2003 (2003-04-01), pages 140-146, XP055205530, ISSN: 1523-3790, DOI: 10.1007/s11912-003-0101-z
- R. HASSAN: "Tumor-Directed Radiation and the Immunotoxin SS1P in the Treatment of Mesothelin-Expressing Tumor Xenografts", CLINICAL CANCER RESEARCH, vol. 12, no. 16, 15 August 2006 (2006-08-15), pages 4983-4988, XP055157621, ISSN: 1078-0432, DOI: 10.1158/1078-0432.CCR-06-0441

### SEQUENCE LISTING

<110> Institut Gustave Roussy
   Centre National de la Recherche scientifique (CNRS)
   Assistance Publique - Hopitaux de Paris (AP-HP)
   Institut Curie
   Université René Descartes - Paris V
   Institut National de la Santé et de la Recherche
   Scientifique (INSERM)
<120> COMBINED VACCINATION/RADIOTERAPY FOR CANCER TREATMENT
<130> STX-B-0001-PCT1
<150> EP 15000507.2
   <151> 2015-02-23
<160> 5
<170> PatentIn version 3.5
<210> 1
   <211> 89
   <212> PRT
   <213> Shigella dysenteriae
<400> 1
   Met Lys Lys Thr Leu Leu Ile Ala Ala Ser Leu Ser Phe Phe Ser Ala
   1               5                   10                  15
   Ser Ala Leu Ala Thr Pro Asp Cys Val Thr Gly Lys Val Glu Tyr Thr
               20                  25                  30
   Lys Tyr Asn Asp Asp Asp Thr Phe Thr Val Lys Val Gly Asp Lys Glu
           35                  40                  45
   Leu Phe Thr Asn Arg Trp Asn Leu Gln Ser Leu Leu Leu Ser Ala Gln
       50                  55                  60
   Ile Thr Gly Met Thr Val Thr Ile Lys Thr Asn Ala Cys His Asn Gly
   65                  70                  75                  80
   Gly Gly Arg Ser Glu Val Ile Phe Arg
                   85
<210> 2
   <211> 69
   <212> PRT
   <213> Artificial Sequence
<220>
   <223> fragment of toxin subunit B from shigella dysenteriae
<400> 2
   Thr Pro Asp Cys Val Thr Gly Lys Val Glu Tyr Thr Lys Tyr Asn Asp
   1               5                   10                  15
   Asp Asp Thr Phe Thr Val Lys Val Gly Asp Lys Glu Leu Phe Thr Asn
               20                  25                  30
   Arg Trp Asn Leu Gln Ser Leu Leu Leu Ser Ala Gln Ile Thr Gly Met
           35                  40                  45
   Thr Val Thr Ile Lys Thr Asn Ala Cys His Asn Gly Gly Gly Arg Ser
       50                  55                  60
   Glu Val Ile Phe Arg
   65
<210> 3
   <211> 15
   <212> PRT
   <213> Artificial Sequence
<220>
   <223> amino acids 43-57 of the HPV 16 E7 protein
<400> 3
   Gly Gln Ala Glu Pro Asp Arg Ala His Tyr Asn Ile Val Thr Phe
   1               5                   10                  15
<210> 4
   <211> 90
   <212> PRT
   <213> Artificial sequence
<220>
   <223> derivative of toxin subunit B from Shigella dysenteriae
<400> 4
   Met Lys Lys Thr Leu Leu Ile Ala Ala Ser Leu Ser Phe Phe Ser Ala
   1 5 10 15
   Ser Ala Leu Ala Thr Pro Asp Cys Val Thr Gly Lys Val Glu Tyr Thr
   20 25 30
   Lys Tyr Asn Asp Asp Asp Thr Phe Thr Val Lys Val Gly Asp Lys Glu
   35 40 45
   Leu Phe Thr Asn Arg Trp Asn Leu Gln Ser Leu Leu Leu Ser Ala Gln
   50 55 60
   Ile Thr Gly Met Thr Val Thr Ile Lys Thr Asn Ala Cys His Asn Gly
   65 70 75 80
   Gly Gly Arg Ser Glu Val Ile Phe Arg Cys
   85 90
<210> 5
   <211> 70
   <212> PRT
   <213> Artificial Sequence
<220>
   <223> derivative of toxin subunit B from Shigella dysenteriae
<400> 5
   Thr Pro Asp Cys Val Thr Gly Lys Val Glu Tyr Thr Lys Tyr Asn Asp
   1 5 10 15
   Asp Asp Thr Phe Thr Val Lys Val Gly Asp Lys Glu Leu Phe Thr Asn
   20 25 30
   Arg Trp Asn Leu Gln Ser Leu Leu Leu Ser Ala Gln Ile Thr Gly Met
   35 40 45
   Thr Val Thr Ile Lys Thr Asn Ala Cys His Asn Gly Gly Gly Arg Ser
   50 55 60
   Glu Val Ile Phe Arg Cys
   65 70

## Description

The present International application claims the priority of the European patent application EP 1 5000507.2 filed on February 23, 2015.

### FIELD OF THE INVENTION:

The present invention relates to the treatment of cancer and more preferably to HPV-positive cancer. The work leading to this invention has received funding from the European Union Seventh Framework Programme (FP7/2007-2013) under grant agreement n° 304810 and from Cancéropole Ile de France (Project HPV-CAN 2011-2013).

### BACKGROUND OF THE INVENTION:

Human Papillomavirus (HPV) is the most common sexually transmitted disease in Western countries. Mucosal infection with HPV types 16 and 18 has long been acknowledged to be a major risk factor for malignant neoplasms such as cervical, anal, penile, vaginal, and head and neck squamous cell carcinoma (HNSCC). The global burden of cancers attributable to HPV infection is estimated at 600,000 cases/year worldwide and accounts for up to 50-80% of oropharyngeal cancers with a steadily increasing rate.

First indications for an involvement of human papillomavirus (HPV) in the etiology of head and neck squamous cell carcinomas (HNSCC) have been obtained in the early 1980s. Subsequently, a specific association of HPV with tumors in Waldeyer's tonsillar ring was found. Research has demonstrated that HPV-positive HNSCC represents a distinct subgroup of malignancies with improved outcomes compared with HPV-negative HNSCC; this is a result of a combination of peculiar biologic features and favorable patient characteristics (younger patients and a minor role of tobacco and alcohol abuse). Since 2000, several larger studies (n>90) confirmed an association between HPV, especially HPV16, and HNSCC, such as oropharyngeal squamous cell carcinomas (OPSCC). Now, both the analysis of viral load and of viral oncogene expression is applied to identify HNSCC with biologically active HPV16, whose oncogenic role is supported by the expression of E6 and E7.

Because of their prognostic, HPV-positive HNSCC patients are more likely to have a durable benefit from radiotherapy, which is now recognized as a valuable option for HNSCC.

Actually, stages I-II HNSCC are treated with a radiation dose depending on tumor size; however, for early stage disease, doses of 66-74 Gy (2.0 Gy/fraction; daily Monday-Friday in 7wk) may be used with adequate results. For later stages III-IV, similar radiotherapy doses are used but in combination with chemotherapy or following surgery.

Because of the increasing incidence of HPV-related cancers and the promising results of radiotherapy, there is a need for the further optimization of radiotherapy treatments by integrating novel strategies to improve radiotherapy's biological efficacy and limit the toxicities related to dose escalation in this subset of patients.

### DETAILED DESCRIPTION OF THE INVENTION:

The inventors established that a combination of a tumor vaccine and of radiotherapy results in a strong synergy leading to the healing of most of the animals, said synergy enabling to halve the radiotherapy dose.

Whereas the inventors established previously that an intranasal immunization with said cancer vaccine was necessary so as to elicit a sustained antitumor response in a murine model of HPV-positive head and neck cancer, they now show that, in combination with radiotherapy, a parenteral administration of said vaccine lead to a strong immune response leading to tumor regression.

The efficiency of said treatment prevents metastasis appearance and also their progression.

Finally, the inventors show that the immunisation with said cancer vaccine may be initiated before irradiation without affecting the efficiency of the treatment.

Thus, in a first aspect, the present invention concerns a composition for use in the prevention or treatment of a subject suffering from a HPV-positive cancer in combination with radiotherapy, wherein said composition comprises a conjugate, eventually associated with pharmaceutically acceptable carrier, said conjugate comprising:
i) the Shiga Toxin B subunit (STxB), a fragment thereof having a length of at least 50 amino acids, which fragment binds to the glycolipid Gb3 receptor with a K_{D} equal or inferior to 10⁻⁷ M or a derivative thereof corresponding to an amino acid sequence binding to the glycolipid Gb3 receptor with a K_{D} equal or inferior to 10⁻⁷ M and having a percentage of identity of at least 95 % as compared to the amino acid SEQ id n°: 1; and
ii) at least one HPV epitope associated with said cancer.

As used herein, the term "subject" denotes a mammal, such as a rodent, a feline, a canine or a primate, and most preferably a human.

As used herein an "epitope associated with a cancer" refers to an epitope derived from an antigen expressed in a cancer..

For a HPV-positive cancer, one may also cite HPV-16 or HPV-18 proteins, such as E6 or E7 proteins.

The Shiga Toxin is a bacterial toxin of the AB₅ subunit family that is secreted by *Shigella dysenteriae.* The A-subunit is the toxic moiety and inhibits the protein synthesis in higher eukaryotic target cells after transferring into the cytoplasm of said cells. The B-subunit is a homopentamer protein (5B - fragments) and is responsible for toxin binding to and internalization into target cells by interacting with the glycolipid Gb3 found on the plasma membranes of these cells. The B-fragment is non toxic, but conserves the intracellular transport characteristics of the holotoxin which, in many Gb3 expressing cells, is transported in a retrograde fashion from the plasma membranes to cytosol, via endosomes. The glycolipid Gb3 receptor enabling said transport, is also reported to be expressed preferentially in some ectodermic derived tumors (plasma) and in some Burkitt's lymphoma, and is also known as CD 77.

As used herein, the term "Shiga Toxin B subunit" or "STxB" corresponds to the polypeptide having the accession number 1410186B and the sequence SEQ id n°1: MKKTLLIAAS LSFFSASALA TPDCVTGKVE YTKYNDDDTF TVKVGDKELF TNRWNLQSLL' LSAQITGMTV TIKTNACHNG GGRSEVIFR

As used herein, the term "fragment" refers to a polypeptide having a length of preferably at least 60 amino acids, and most preferably of at least 65 amino acids, which fragment binds preferably to the glycolipid Gb3 receptor with a K_{D} equal or inferior to 10⁻⁸ M. Preferably said STxB fragment refers to the sequence SEQ id n°2: TPDCVTGKVE YTKYNDDDTF TVKVGDKELF TNRWNLQSLL LSAQITGMTV TIKTNACHNG GGRSEVIFR

As used herein, the term "STxB derivatives" refers to an amino acid sequence which binds preferably to the glycolipid Gb3 receptor with a K_{D} equal or inferior to 10⁻⁸ M, and which has preferably a percentage of identity of at least 97.5 % (i.e. corresponding to about 2 amino acids substitutions) as compared to the amino acid SEQ id n°: 1,), and more preferably of at least 98.5% (i.e. corresponding to about 1 amino acids substitutions). Such derivatives can be simply identified by the skilled person in view of its personal knowledge and of the teaching of the present patent application. It will also be understood that natural amino acids may be replaced by chemically modified amino acids. Typically, such chemically modified amino acids increase the polypeptide half life.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developed by NEDDLEMAN and WUNSCH (J. Mol. Biol., vol.48, p:443, 1970), by using the method of similarities developed by PEARSON and LIPMAN (Proc. Natl. Acad. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p:1792, 2004). To get the best local alignment, one can preferably use the BLAST software with the BLOSUM 62 matrix. The identity percentage between two sequences of amino acids is determined by comparing these two sequences optimally aligned, the amino acids sequences being able to encompass additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

In a preferred embodiment, the HPV epitope is a HPV16 epitope.

As used herein, the term "HPV-positive cancer" refers to a malignant neoplasm associated with a HPV infection, and thus expressing HPV antigen. As an example, such malignant neoplasms may include cervical, anal, penile, vaginal, and head and neck squamous cell carcinoma (HNSCC). Preferably, said HPV-positive cancer is a HPV-positive HNSCC.

Now, said cancer may also correspond to metastasis.

Thus, said HPV-positive cancer may be a metastasis.

As used herein, the term a "HPV16 epitope" refers to HPV16 peptidic sequence, which can be presented by MHC class II. Preferably, said HPV16 epitope is a HPV16 epitope from the E6 or E7 protein of HPV16. The sequence of the E6 protein from HPV16 is well known from the skilled person. As an example, one can cite the sequence of E6 protein with the accession numbers AHK23256.1, AIQ82784.1, AGM34407.1, ACJ66714.1 or AAO85408.1. The sequence of the E7 protein from HPV16 is also well known from the skilled person. As an example, one can cite the sequence of E7 protein with the accession numbers AHK23257.1, AIQ82815.1, AGM34442.1, ACR25131.1 or ACJ66717.1.

The skilled person can also simply identify the HPV16 epitope among E6 or E7 protein. As an example, one can cite those disclosed in WO 02/090382 or WO2008147187 disclosing E6 and E7 HPV16 epitopes, or in WO 02/070006 disclosing the peptide consisting of amino acids 127-142 of HPV16 E6 protein, the peptide consisting of amino acids 35-50 of HPV 16 E7 protein, the peptide consisting of amino acids 43-77 of HPV16 E7 protein or the peptide consisting of amino acids 50-62 of HPV 16 E7 protein. One can also cites the epitope disclosed in STRANG et al. (J Gen. Virol., vol.71, p:423-31, 1990) corresponding to a peptide consisting of amino acids 42-57 of HPV16 E6 protein, or the one disclosed in ALTMANN et al. (Eur. J. Cancer, vol.28, p:326-33, 1992) corresponding to the peptide consisting of amino acids 5-18 of HPV16 E7 protein, the peptide consisting of amino acids 17-34 of HPV 16 E7 protein and the peptide consisting of amino acids 69-82 of HPV 16 E7 protein. One can also cites the peptide consisting of amino acids 43-57 or 49-57 of HPV16 E7 protein disclosed in ADOTEVI et al. (J. Immunol., vol., p: 3371-3379, 2007).

Advantageously, said HPV16 epitope is from HPV16 E7 protein, and is selected in the group comprising the peptides consisting of amino acids 5-18. 17-34, 35-50, 43-57, 43-77, 49-57, 50-62, 69-82 of the HPV 16 E7 protein. Preferably, said HPV16 epitope consists in the amino acids 43-57 of the HPV 16 E7 protein (SEQ id n°3; GQAEPDRAHYNIVTF).

The conjugate comprises a pentamer of STxB, fragment or derivative thereof. In this conjugate, i) the Shiga Toxin B subunit (STxB), a fragment or a derivative thereof; and ii) the at least one HPV16 epitope may be also covalently linked using bifunctional protein coupling agents or in a fusion protein. Naturally, the natural amino acids in said conjugate may be replaced by chemically modified amino acids. Typically, such chemically modified amino acids enable to increase the polypeptide half-life.

In a particular embodiment, i) the Shiga Toxin B subunit (STxB), a fragment or a derivative thereof; and ii) the at least one cancer associated epitope are covalently linked using a bifunctional protein coupling agents.

Bifunctional protein coupling agents are well known from the skilled person such as methods using them, and include, as examples, m-maleimidobenzoyl-N-hydroxysulfosuccinimide ester, N-succinimidyl (2-pyridyldithio) propionate (SPDP), succinimidyl (N-maleimidomethyl) cyclohexane-1-carboxylate, iminothiolane (IT), bifunctional derivatives of imidoesters (such as dimethyl adipimidateHCL), active esters (such as disuccinimidylsuberate), aldehydes (such as glutaraldehyde), bis-azido compounds (such as bis (p-azidobenzoyl) hexanediamine), bis-diazonium derivatives (such as bis-(p-diazoniumbenzoyl)-ethytenediamine), diisocyanates (such as toluene 2,6diisocyanate), and bis-active fluorine compounds (such as 1,5-difluoro-2,4- dinitrobenzene).

Preferably, the i) the Shiga Toxin B subunit (STxB), a fragment or a derivative thereof refers to the sequences disclosed in WO 02/060937, wherein a Cysteine residue is added at the C-terminus. As an example, said STxB derivatives refer to the SEQ id n°4 and SEQ id n°5, preferably SEQ id n°5.

| | |
|---|---|
| SEQ id n°4 | |
| SEQ id n°5 | |

In a particular embodiment, i) the Shiga Toxin B subunit (STxB), a fragment or a derivative thereof; and ii) the at least one cancer associated epitope are covalently linked in a fusion protein.

The term "fusion protein", also known as a chimeric protein, refers to a protein created through the joining of i) the Shiga Toxin B subunit (STxB), a fragment or a derivative thereof; and ii) the at least one cancer associated epitope, which are originally coded for separate polypeptides.

Both sequences may be separated or not by a "linker" amino acid sequence.

In a particular embodiment, the sequences i) and ii) are not separated by any inker such as for the conjugate disclosed in WO 99/03881. and Haicheur et al (J Immunology vol.165(6) p.3301-3308 2000).

Said "linker" amino acid sequence may be of a length sufficient to ensure that the fusion protein form proper secondary and tertiary structures. The length of the linker amino acid sequence may vary without significantly affecting the biological activity of the fusion protein. Typically, the length of the linker is comprised between 5 and 30 amino acids, preferably between 10 and 30 amino acids, more preferably between 15 and 30 amino acids.

Preferred linker amino acid sequences are those which allow the conjugate to adopt a proper conformation. The most suitable linker amino acid sequences (1) will adopt a flexible extended conformation, (2) will not exhibit a propensity for developing ordered secondary structure which could interact with the functional domains of fusion proteins, and (3) will have minimal hydrophobic or charged character which could promote interaction with the functional protein domains. Preferably, the linker amino acid sequence comprises near neutral amino acids selected in the group comprising Gly (G), Asn (N), Ser (S), Thr (T), Ala (A), Leu (L), and Gln (Q), most preferably in the group comprising Gly (G), Asn (N), and Ser (S). Examples of linker sequences are described in U.S. Pat. Nos. 5,073,627 and 5,108,910.

The composition comprises an "effective amount" of the conjugate, which effective amount is sufficient to induce an immune response against the at least one cancer associated epitope, preferably sufficient to induce the regression of tumor growth, notably in combination with radiotherapy. The doses used for the administration can be adapted as a function of various parameters, in particular as a function of the mode of administration used, of the relevant pathology, or alternatively of the desired duration of treatment. Naturally, the form of the pharmaceutical composition, the route of administration, the dosage and the regimen naturally depend on the condition to be treated, the severity of the illness, the age, weight, and sex of the subject, etc. The ranges of effective doses provided below are not intended to limit the invention and represent preferred dose ranges. However, the preferred dose can be tailored to the individual subject, as is understood and determinable by one of skill in the art, without undue experimentation.

In view of the marked efficiency of said conjugate, the skilled person can plan to use small doses for treating a subject suffering from a cancer. As a non limiting example, the conjugate can be parenterally administered by injection at a dose in a range between 10 µg and 1000 µg, preferably in a range between 100 µg and 300 µg Now, said conjugate administration may be done once, or repeated one or several time during the treatment of the subject.

Radiation therapy or radiotherapy is the medical use of irradiation -i.e. ionizing radiation- as part of cancer treatment to control malignant cells. It is used as palliative treatment or as therapeutic treatment. Radiotherapy is accepted as an important standard therapy for treating various types of cancers. It is known that vaccine or antibody therapy can be combined with radiotherapy as for example show in WO2012/177624 A2.

As used herein, the term "radiotherapy" is used for the treatment of diseases of oncological nature with irradiation corresponding to ionizing radiation. Ionizing radiation deposits energy that injures or destroys cells in the area being treated (the target tissue) by damaging their genetic material, making it impossible for these cells to continue to grow. Radiotherapy may be used to treat localized solid tumors cancers of the skin, tongue, larynx, brain, breast, lung or uterine cervix. One type of radiation therapy commonly used involves photons, e.g. X-rays. Depending on the amount of energy they possess, the rays can be used to destroy cancer cells on the surface of or deeper in the body. The higher the energy of the x-ray beam, the deeper the x-rays can go into the target tissue. Linear accelerators and betatrons produce x-rays of increasingly greater energy. The use of machines to focus radiation (such as x-rays) on a cancer site is called external beam radiotherapy. Gamma rays are another form of photons used in radiotherapy. Gamma rays are produced spontaneously as certain elements (such as radium, uranium, and cobalt 60) release radiation as they decompose, or decay.

Another technique for delivering radiation to cancer cells is to place radioactive implants directly in a tumor or body cavity. This is called internal radiotherapy.

Brachytherapy, interstitial irradiation, and intracavitary irradiation are types of internal radiotherapy. In this treatment, the radiation dose is concentrated in a small area, and the patient stays in the hospital for a few days. Internal radiotherapy is frequently used for cancers of the tongue, uterus, and cervix.

A further technique is intra-operative irradiation, in which a large dose of external radiation is directed at the tumor and surrounding tissue during surgery.

Another approach is particle beam radiation therapy. This type of therapy differs from photon radiotherapy in that it involves the use of fast-moving subatomic particles to treat localized cancers. Some particles (neutrons, pions, and heavy ions) deposit more energy along the path they take through tissue than do x-rays or gamma rays, thus causing more damage to the cells they hit. This type of radiation is often referred to as high linear energy transfer (high LET) radiation. Radio-sensitizers make the tumor cells more likely to be damaged, and radio-protectors protect normal tissues from the effects of radiation.

A person of ordinary skill in the radiotherapy art knows how to determine an appropriate dosing and application schedule, depending on the nature of the disease and the constitution of the patient. In particular, the person knows how to assess dose-limiting toxicity (DLT) and how to determine the maximum tolerated dose (MTD) accordingly.

More particularly, the amount of radiation used in photon radiation therapy is measured in gray (Gy), and varies depending on the type and stage of cancer being treated. For curative cases, the typical dose for a solid epithelial tumor ranges from 60 to 80 Gy. Many other factors are considered by radiation oncologists when selecting a dose, including whether the patient is receiving chemotherapy, patient co-morbidities, whether radiation therapy is being administered before or after surgery, and the degree of success of surgery. Moreover, the total dose is often fractionated (spread out over time) for several important reasons. In fact, fractionation allows normal cells time to recover, while tumor cells are generally less efficient in repair between fractions. Fractionation also allows tumor cells that were in a relatively radioresistant phase of the cell cycle during one treatment to cycle into a sensitive phase of the cycle before the next fraction is given. Similarly, tumor cells that were chronically or acutely hypoxic (and therefore more radioresistant) may reoxygenate between fractions, improving the tumor cell kill. Fractionation regimes are individualized between different radiotherapy centers and even between individual doctors. In North America, Australia, and Europe, the typical fractionation schedule for adults is 1.8 to 2 Gy per day, five days a week. In some cancer types, prolongation of the fraction schedule over too long can allow for the tumor to begin repopulating, and for these tumor types, including head-and-neck and cervical squamous cell cancers, radiation treatment is preferably completed within a certain amount of time. In some cases, two fractions per day are used near the end of a course of treatment. This schedule, known as a concomitant boost regimen or hyperfractionation, is used on tumors that regenerate more quickly when they are smaller. In particular, tumors in the head-and-neck demonstrate this behavior.

The inventors have established that the combined treatment with the conjugate and radiotherapy enables to halve the radiotherapy doses for obtaining a healing.

Thus, in another preferred embodiment, and because of the efficiency of the method of the invention, the radiotherapy can be applied at a dose in a range from about 10 to 55Gy, preferably from about 15 to 50 Gy, such as 20 to 40Gy, , concretely from about 20 to 35 Gy, and more concretely from about 25 to 30 Gy.

As used herein, the term "in combination" refers to the use of more than one therapeutic agent, radiotherapy and the conjugate in the present case. The use of the term "in combination" does not restrict the order in which said therapeutic agents are administered to the subject. A first therapeutic agent, such as a conjugate, can be administered prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of radiotherapy, to a subject with cancer.

Thus, the composition and the radiotherapy may be administrated currently, separately, or sequentially.

The inventors have established that the combination is very efficient by administrating the first dose of the vaccine conjugate prior irradiation. This approach will facilitate the implementation of vaccinations in future clinical strategies since patients with a confirmed diagnosis of HPV-positive cancer would immediately benefit from the vaccination administration while waiting for radiotherapy treatment.

Thus, in a third preferred embodiment, the composition is administrated before the irradiation, preferably at a timing of 1 minute to 14 days before irradiation, most preferably 3 to 7 days before irradiation. Accordingly, said composition is administrated in a subject who has not received radiotherapy, preferably who has not received irradiation since at least 1 minute to 14 days, preferably at least 1 to 3 days.

The expression "pharmaceutically acceptable" refers to molecular entities and compositions that are physiologically tolerable and do not typically produce allergic or similar undesirable reactions, such as gastric upset, dizziness and the like when administered to a human. Preferably, as used herein, the expression "pharmaceutically acceptable" means approvable by a regulatory agency of the Federal or state government or listed in the U.S. Pharmacopeia or other generally recognized pharmacopeia for use in animals, and more particularly in humans.

The term "carrier" refers to a solvent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like.

The conjugate may be solubilized in a buffer or water or incorporated in emulsions, microemulsions, hydrogels (e.g. PLGA-PEG-PLGA triblock copolymers-based hydrogels), in microspheres, in nanospheres, in microparticles, in nanoparticles (e.g. poly(lactic-co-glycolic acid) microparticles (e.g. poly lactic acid (PLA) ; poly (lactide-co-glycolic acid) (PLGA) ; polyglutamate microspheres, nanospheres, microparticles or nanoparticles), in liposomes, or other galenic formulations. In all cases, the formulation must be sterile and fluid to the extent of acceptable syringability. It must be stable under the conditions of manufacture and storage and must be preserved against the contaminating action of microorganisms, such as bacteria and fungi.

Dispersions can also be prepared in glycerol, liquid polyethylene glycols, and mixtures thereof and in oils. Under ordinary conditions of storage and use, these preparations contain a preservative to prevent the growth of microorganisms.

The conjugate can be formulated into a composition in a neutral or salt form. Pharmaceutically acceptable salts include the acid addition salts (formed with the free amino groups of the protein) which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or a dispersion medium containing, for example, water, ethanol, polyol (for example, glycerol, propylene glycol, and liquid polyethylene glycol, and the like), suitable mixtures thereof, and vegetables oils. The conjugates of the invention may also be modified, by pegylation as an example, so as to increase its biodisponibility.

The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

Prolonged absorption of the injectable compositions can be brought about by the use in the compositions of agents delaying absorption, for example, aluminium monostearate, gelatin, polyols, and half-life enhancing covalent and non covalent formulations.

There are numerous causes of peptide instability or degradation, including hydrolysis and denaturation. Hydrophobic interaction may cause clumping of molecules together (i.e. aggregation). Stabilizers may be added to reduce or prevent such problems.

Stabilizers include cyclodextrine and derivatives thereof (see U.S. Pat. No.5,730,969). Suitable preservatives such as sucrose, mannitol, sorbitol, trehalose, dextran and glycerin can also be added to stabilize the final formulation. A stabilizer selected from ionic and non-ionic surfactants, D-glucose, D-galactose, D-xylose, D-galacturonic acid, trehalose, dextrans, hydroxyethyl starches, and mixtures thereof may be added to the formulation. Addition of alkali metal salt or magnesium chloride may stabilize a peptide. The peptide may also be stabilized by contacting it with a saccharide selected from the group consisting of dextran, chondroitin sulphuric acid, starch, glycogen, dextrin, and alginic acid salt. Other sugars that can be added include monosaccharides, disaccharides, sugar alcohols, and mixtures thereof (E.g., glucose, mannose, galactose, fructose, sucrose, maltose, lactose, mannitol, xylitol). Polyols may stabilize a peptide, and are water-miscible or water-soluble. Suitable polyols may be polyhydroxy alcohols, monosaccharides and disaccharides including mannitol, glycerol, ethylene glycol, propylene glycol, trimethyl glycol, vinyl pyrrolidone, glucose, fructose, arabinose, mannose, maltose, sucrose, and polymers thereof. Various excipients may also stabilize peptides, including serum albumin, amino acids, heparin, fatty acids and phospholipids, surfactants, metals, polyols, reducing agents, metal chelating agents, polyvinyl pyrrolidone, hydrolysed gelatin, and ammonium sulfate.

The composition may be formulated for injection, e.g. local injection, transmucosal administration (i.e. intranasal...), inhalation, oral administration and more generally any formulation that the skilled person finds appropriate to achieve the desired therapy.

Now, whereas the inventors have priory established that the HPV vaccine induces sufficient immune response only by intranasal administration; they now show that a parenteral administration in combination with radiotherapy induces a sufficient immune response so as to destroy the target cancer.

Thus, in still another preferred embodiment, the composition is for use in the prevention or treatment of a subject suffering from a cancer attributable to HPV infection by a parenteral administration.

As used herein, the term "parenteral" includes intravenous, intramuscular, subcutaneous, rectal, vaginal or intraperitoneal administration.

Thus, the used composition contains vehicles which are pharmaceutically acceptable for a formulation intended to be injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. Suitable pharmaceutical carriers are described in "Remington's Pharmaceutical Sciences" by E.W. Martin.

Other embodiments and advantages of the present invention are illustrated in the following examples.

### EXAMPLES

### 1) StTxB-E7 Vaccine Production:

The StTxB-E7 vaccine was produced by the chemical coupling of the N-bromoacetylated E7₄₃₋₅₇ peptide (SEQ id n°...; GQAEPDRAHYNIVTF) and the sulfhydryl group of the recombinant nontoxic B subunit of Shiga toxin (SEQ id n°...) as disclosed in HIACHEUR et al. (Journal of immunology, vol. 165(6), p:3301-8, 2000).

### 2) Head and neck tumor and lung metastasis models

Female C57BL/6 mice (age, 7 to 8 weeks) were purchased from JANVIER CERT and housed in the GUSTAVE ROUSSY animal facility. All animal procedures were performed according to the protocols approved by the Ethical Committee CEEA 26 and in accordance with recommendations for the proper use and care of laboratory animals.

To establish a head and neck tumor implantation model, TCl/Luc cells were used. These firefly luciferase-expressing TCl/Luc cells, generated by the HPV16 E6/E7 and c-H-ras retroviral transduction of lung epithelial cells of C57BL/6 origin, were kindly provided by T.C. WU (Johns Hopkins Medical Institutions, Baltimore, MD, USA). The cells were cultured *in vitro* in RPMI 1640 supplemented with 10% fetal bovine serum, 1% sodium pyruvate, 1% non-essential amino acids and 10 mM HEPES and were grown at 37°C with 5% CO2. Then, syngeneic tumor grafts were initiated by injection (Day 0) of 0.1 ml of PBS suspension containing 5× 10⁵ TCl/Luc cells at a submucosal site of the right inner lip of the mice. For rechallenge experiments, mice were injected in the left inner lip. Lung metastases were established by injecting 10⁶ TCl/Luc cells suspended in 0.2 ml of PBS in the tail vein.

### 3) Radiotherapy potentialization using STxB-E7 vaccine

The mice were then subjected to the therapeutic treatments depicted in the schematic of figure 1, with 10 mice per group. Briefly, the mucosal adjuvant α-galactosylceramide (KRN7000, FUNAKOSHI, 20 µg per mouse) was administered intraperitonealy at Day 3 alone or together with the first dose of vaccine STxB-E7 (1 µg per mouse). Then, the mice received or not a single-beam local irradiation to the head and neck region using a 200 kV Varian X-ray irradiator. This selective irradiation of the tumor grafts was performed by the interposition of a 4-cm-thick lead shield and corresponded to a single fraction 7.5 Gy. The KRN7000-treated mice were used as control groups since KRN7000, either alone or in combination with radiotherapy, did not impact tumor growth (data not shown).

To monitor tumor growth, bioluminescence imaging was performed using the XENOGEN *IN VIVO* IMAGING SYSTEM 50 (IVIS; CALIPER LIFE SCIENCES). Mice were placed in the supine position in the imaging chamber 5 minutes after intraperitoneal injection of firefly luciferin; images were acquired over a 15- to 30-second period while the mice were under sedation via the continuous administration of isoflurane. The photographic images and bioluminescence color images were superimposed, and signal quantification was performed using the LIVINGIMAGE V 4.3.1 software (CALIPER LIFE SCIENCES). The bioluminescent signal as a measure of tumor size was analyzed using an ANOVA analysis followed by Tukey's post-hoc test for multiple comparisons. Simultaneously, the survival data were analyzed using the Kaplan-Meier and Log-rank tests for survival distribution. Correlations were assessed using Pearson's correlation test, and statistical analyses were performed using PRISM Version 6 (GRAPHPAD).

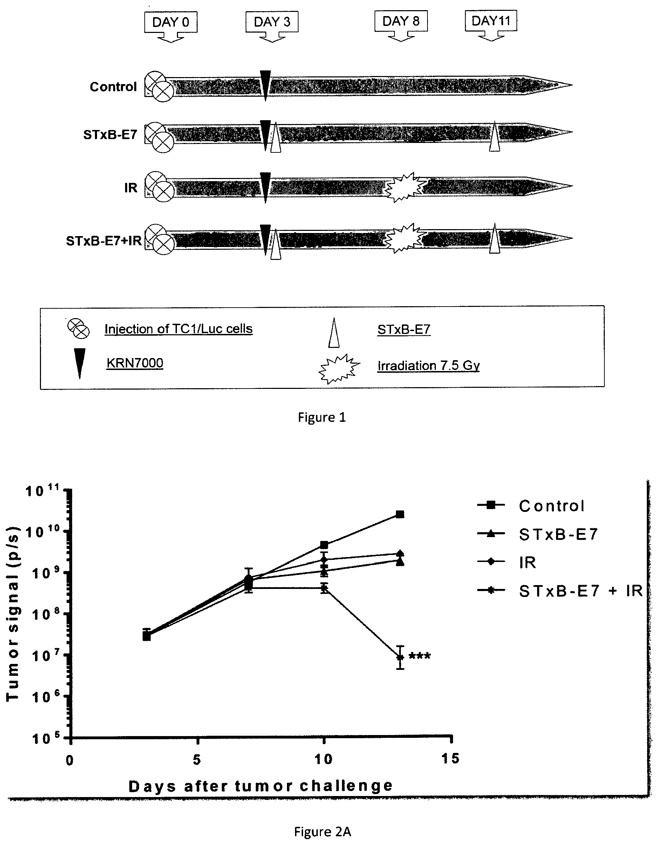

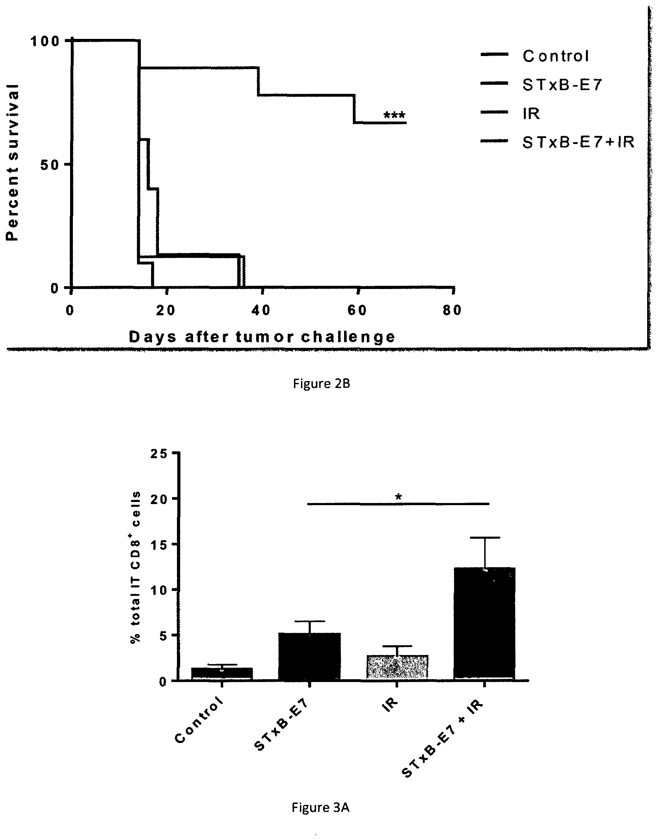

The figure 2A shows the tumor signal as determined by in *vivo* bioluminescent imaging following TCl/Luc cells mouse injection with time, and the figure 2B shows (***p<0.001) the Kaplan-Meier survival curves for the different groups.

The bioluminescent *in vivo* imaging results showed that the mice developed fast-growing tumors (Fig. 2A) that resulted in the survival of control mice for only two weeks after TCl/Luc cell injection (Fig. 2B). Treatment with a single dose of 7.5 Gy IR resulted in a limited tumor growth delay and a non-significant enhanced survival (Fig. 2B).

The systemic immunization with STxB-E7 only partially increased tumor control, whereas its combination with local radiotherapy led to an effective antitumor response with 70% of the mice still alive 70 days after the tumor graft (Fig. 2B, p<0.001 *vs* control). Moreover, the tumor volume of mice treated with the combined therapy was significantly smaller compared with the one of single therapy (IR or STxB-E7)-treated mice (Fig. 2A, p<0.01, one-way ANOVA). Finally, most of the mice in the combination therapy group had complete tumor clearance that lasted until the end of the 90-day observation period (data not shown). Of note, when the tumors were irradiated with a 10.4 Gy dose in 4 consecutive fractions (2.6 Gy/day), representing the biologically equivalent dose (BED) of 7.5 Gy in a single fraction as calculated using the linear quadratic model with an α/β=10, the results were similar (data not shown).

These data indicate that the novel therapeutic approach based on the combination of STxB-E7 with radiotherapy resulted in the complete clearance of the majority of treated tumors in a relevant model of HNSCC, even after an 18-month observation period. This new radiovaccination strategy acts synergistically with either single dose or fractionated IR, which is of particular relevance because fractionated radiotherapy is the mainstay for the treatment of HNSCC. Moreover, the possibility to use low irradiation doses could be a valuable tool for treatment deintensification strategies in the clinic, since it can limit the risk of late complications.

### 4) tumor-infiltrating and splenic antigen-specific CD8⁺ T cells induced by combined irradiation and vaccination

Since CD8⁺ T cell population play a pivotal role in the tumor control of TCI, the presence of anti- E7₄₉₋₅₇/D^{b}-specific CD8⁺ T cells in tumors was analyzed.

For this analysis, 4 groups of 5 mice were treated as described previously and their tumors and spleen were then dissociated at day 14, washed twice in PBS and then incubated with the Fc receptor block CD16/CD32 (EBIOSCIENCE). The tumors were then labeled with the E7₄₉₋₅₇/D^{b} tetramer according to the manufacturer's recommendations (BECKMAN COULTER IMMUNOMICS) and the anti-CD8 antibody. Cells were incubated for 35 minutes at 4°C with the PE-labeled tetramer. After incubation and washes, the cells were labeled using the Live/Dead cell viability assay (LIFE TECHNOLOGIES) and anti-CD8 (APC-Cy7, EBIOSCIENCE), PDI (FITC, EBIOSCIENCE), CTLA4 (Brilliant Violet 421, BIOLEGEND), and Tim3 (APC, EBIOSCIENCE) mAbs for 20 minutes to phenotype the tetramer-positive CD8⁺ T cells. Irrelevant tetramers recognizing a LCMV derived peptide in the context of D^{b} molecules were used. CD8⁺ cells were detected by flow cytometry and expressed as percentage of total living cells using the FLOWJO (TREE STAR) software.

The figures 3A and 3B show the E7-specific CD8⁺ T cells detected intratumorally as expressed as the percentage of total CD8+ cells and the percentage of H-2Db E7-tetramer per CD8⁺T cells.

The figure 4 shows percentage of H-2Db E7-tetramer per CD8⁺T cells in the spleen of said mice.

The figure 5 shows the tumor sizes obtained by IVIS *in vivo* imaging at the day of sacrifice plotted against the percentage of tumor infiltrating E7-specific CD8⁺ *p<0.05; one-way ANOVA followed by Tukey's post-test.

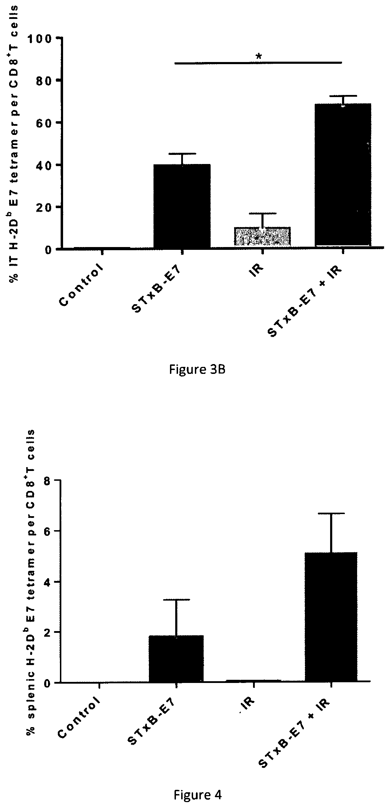

The results show that the total intratumor CD8⁺ levels were low in control tumors, and almost no E7-specific CD8⁺ T cells were found in this group (Fig. 3). Irradiation alone was not sufficient to alter the CD8⁺ infiltrate, whereas vaccination upregulated the percentage of E7₄₉₋₅₇ specific CD8⁺ T cells, as demonstrated by the E7-tetramer analysis. When IR was added to STxB-E7 immunization, the infiltration of CD8⁺ T cells was significantly boosted (Fig. 3A). Most of the CD8⁺ T cells were antigen-specific, as the level of E7 tetramer-positive CD8⁺ T lymphocytes reached a surprising 67% in this group (Fig. 3B).

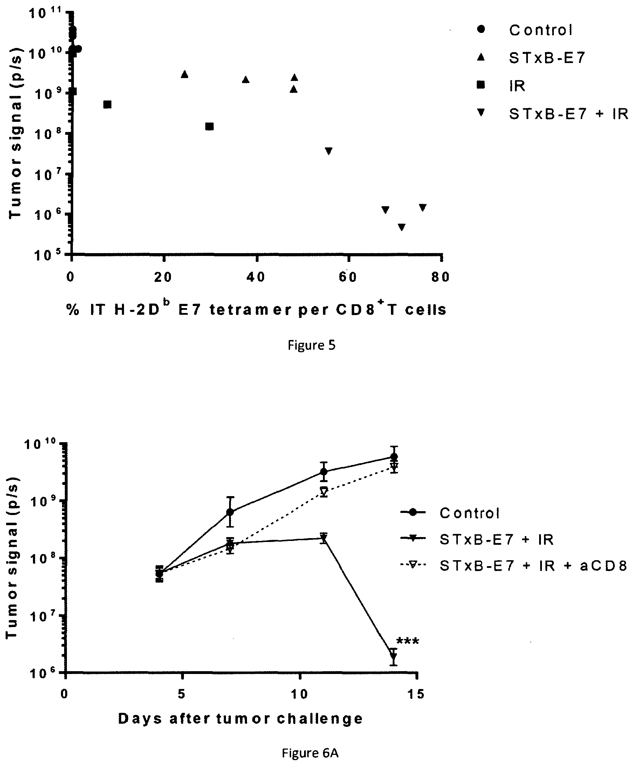

The qualitative analysis of the E7-specific CD8⁺ infiltrate by identifying the PD1, CTLA4 and Tim3 positive subpopulations does not reveal any significant differences among the treatment groups (data not shown). Now, the induction of a specific CD8⁺ T cell response was not restricted to the tumor but was instead systemic. Indeed, the combination of IR and vaccination resulted in the highest antigen-directed response compared with vaccine treatment alone (Fig. 4). Moreover, the level of E7-specific CD8⁺ lymphocytes was inversely correlated with the tumor size (p<0.05, Pearson's correlation test, Fig. 5), suggesting a central role of CD8⁺ T lymphocytes in the antitumor response in the preclinical HNSCC tumor model.

To mechanistically validate the role of CD8⁺ T cells in this therapeutic setting, mice undergoing combined irradiation and vaccination were injected intraperitonealy with 100 µg per mouse of anti-CD8 mAb (clone 2.43; BIOXCELL) or isotype control mAb on day 7 (one day prior to irradiation) to deplete CD8⁺ T cells, as previously reported (SANDOVAL et al., Science translational medicine, vol.5(172), p:172ra20, 2013).

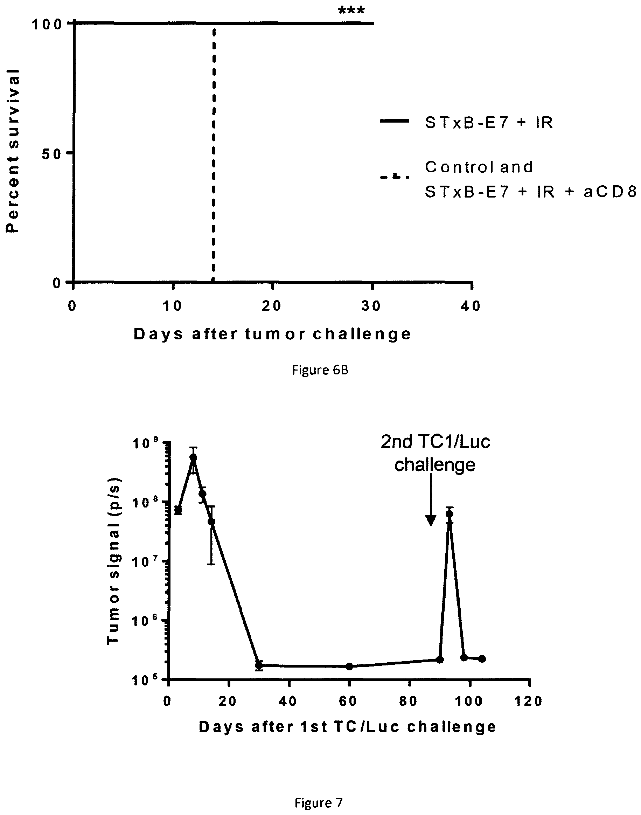

The figures 6A and B show the *in* vivo bioluminescent imaging and the Kaplan curves respectively the mice (N=5) injected with anti-CD8 mAb or isotype control mAb on day 7 (one day prior to irradiation) to deplete CD8⁺ T cells (***p<0.001, one-way ANOVA followed by Tukey's post-test of tumor sizes at day 14). One of two representative experiments is shown.

The results show that the administration of anti-CD8 mAbs has a dramatic effect on the antitumor response, resulting in a complete loss of efficacy of the combined treatment on tumor size and survival, as all of the CD8⁺-depleted mice had to be euthanized together with the controls (Fig. 6A and B).

These data clearly indicate that CD8⁺ T lymphocytes are required to elicit the antitumor response triggered by the combination of irradiation and vaccination.

### 5) T cell memory and STxB-E7/Radiotherapy combination:

Because most of the mice that received the combination of STxB-E7 and IR. surprisingly showed a complete remission of the tumor (Fig. 2B), it was wondered whether the sustained CD8⁺ T cell response observed in this group resulted in the development of an effective T cell memory.

To test this hypothesis, four mice that were still tumor free 90 days after tumor grafting, as observed by *in vivo* imaging as previously, were challenged again with TCl/Luc cells in the submucosa of the opposite inner lip; the opposite side was used to avoid potential bias due to local response. It was confirmed that four days after the second injection, all mice were correctly engrafted with tumor cells localized on left inner lip.

The figure 7 shows the results of *in vivo* bioluminescent imaging establishing the tumor signal following the first and second TCl/Luc challenge.

The results show that five days following the second TCl/Luc challenge, and without any additional treatment, the luciferase activity was completely absent, indicating a spontaneous clearance of the tumor cells. In contrast, treatment-naive mice used as positive controls displayed a tumor growth curve similar to those depicted previously (data not shown). When mice were euthanized to collect the spleens 14 days after the second TCl/Luc challenge, the same results were observed (data not shown). Cytofluorimetric analysis showed that rechallenged mice displayed high percentages of E7-specific CD8⁺ T cells at similar levels to those of mice at the end of the combined treatment (data not shown). Finally, a similar experiment was performed on three mice that were rechallenged with TCl/Luc cells 18 months after the first treatment, again resulting in the complete spontaneous rejection of tumor cells (data not shown).

These data indicate that a combination of local irradiation and immunization elicits a CD8⁺ T cell memory that is sufficient to exert a complete antitumor response.

This strong antitumor response may also be explained by the fact that in contrast to other studies, the combination of low doses of irradiation and the cancer vaccine used here did not result in an increase in immunosuppressive cells in the tumor microenvironment (data not shown).

### 6) Metastasis formation and STxB-E7/Radiotherapy combination:

It was also important to evaluate whether the immune response elicited from effective combined therapy provided protection against potential metastasis formation.

This was tested this by intravenously injecting TCl/Luc cells to induce the development of lung metastases. The metastasis presence was confirmed by *in vivo* imaging analysis showing that, in treatment-naive mice, a bioluminescent signal localized to the lungs was already detectable four days after the injection of TCl/Luc cells (data not shown). The size of the lung metastases increased quickly until day 15, when the mice had to be euthanized.

For this evaluation, the results have shown that, in mice that had already experienced a complete remission of the head and neck tumor upon administration of the combination of STxB-E7 and IR (rechallenge group), the tumor signal in the lungs was already smaller at day 4 when compared to treatment-naïve mice. TCl/Luc cells were spontaneously cleared starting at day 8 and all four tested mice remained metastasis-free until the end of the 60-day observation period (data not shown).

These data indicate that the combination of local irradiation and immunization is sufficient to exert a complete antitumor response not only on local recurrences, but also on distant metastases.

Consequently, this combination strategy represents a promising clinical tool, since a patient treated with this combination therapy who undergoes tumor remission will likely be protected against any local or distant relapse.

### 7) Tumor vessel remodelling and tumor perfusion

The tumor vascular structure and its activation were assessed in the different treatment groups by immunohistochemistry (IHC). For this, tumors were sampled 9 or 14 days after irradiation. These tumors were then included in OCT for further analysis.

Then, detection on tumor slides of alpha smooth muscle actin (αSMA) was performed using a validated standard protocol on a VENTANA DISCOVERY ULTRA autostainer (ROCHE TISSUE DIAGNOSTICS, primary Ab ref ab5694, ABCAM) according to manufacturers' instructions. Manual staining protocols on OCT frozen sections were used to detect CD31 (BD PHARMINGEN), and ICAM-1 (ABCAM). All the slides were counterstained with hematoxylin. NG2 (primary Ab: MILLIPORE) was detected by immunofluorescence. CD31 and αSMA staining were then quantified using the algorithm for vessel detection in the Tissue Studio software package from DEFINIENS. Vessels were automatically detected according to their IHC staining spectral properties in regions of interest that had been manually selected. The percentage of the area stained for NG2 was measured using the IMAGEJ V1.41 software. ICAM1 staining was determined using a semi-quantitative score based on the staining area (less than 1 per field=0; 1-2 per field=1; 3 or more per field=2) and intensity (no staining=0; weak=l; moderate=2; strong=3).

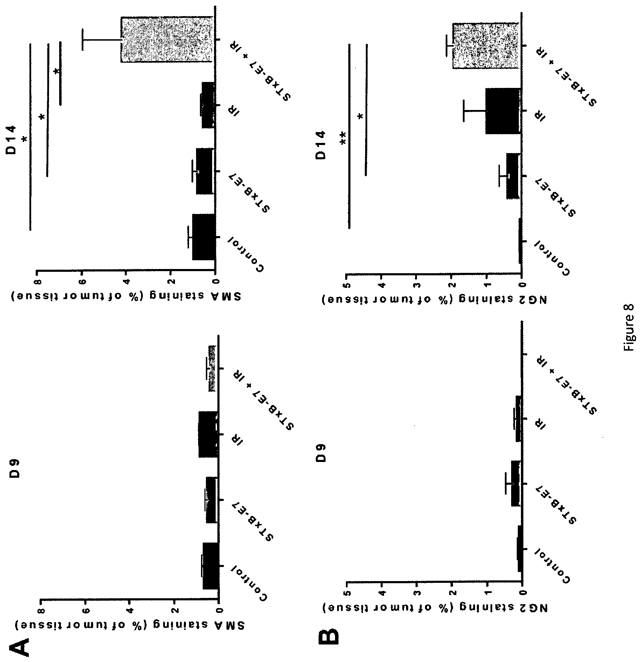

The figure 8 shows the quantification of the antibody stained area for αSMA (A) or NG2 (B) in the tumor sampled at day 9 (D9) or 14 (D14). *p<0.05; **p<0.01 one-way ANOVA followed by Tukey's post-test.

In view of the immunohistochemical staining obtained with CD31, the results established that there were no significant differences in tumor vessel surface and density elicited by the various treatments at either 24 h (day 9) or 6 days after irradiation (day 14) data not shown). In contrast, when the levels of pericytes were analyzed by performing αSMA staining, a significant increase in the stained surface in the tumors of mice treated with both STxB-E7 and IR we observed at day 14 (Fig. 8A). Because pericytes are recognized to be key regulators of the vascular structure and because the extent of their coverage on tumor vessels is typically diminished compared with normal tissues, these data suggest that the combined treatment induces a restoration of vascular functionality. This hypothesis was further confirmed by the increase in NG2 staining (an alternative pericyte marker) observed at day 14 in mice treated with both local irradiation and vaccination (Fig. 8B).

To assess vascular permeability, 0.2 ml Evans Blue dye (0.5%) was administered to mice by tail vein injection. Mice were sacrificed 30 minutes after said injection. Dissected tumor samples were collected, and Evans Blue was eluted in formaldehyde (0.5 ml for 50 mg of tissue) at 58° C overnight. The concentration of the accumulated dye for each aliquot was quantified by the use of a spectrophotometer by reading the absorbance at 610 nm with subtraction of the reference absorbance at 450 nm.

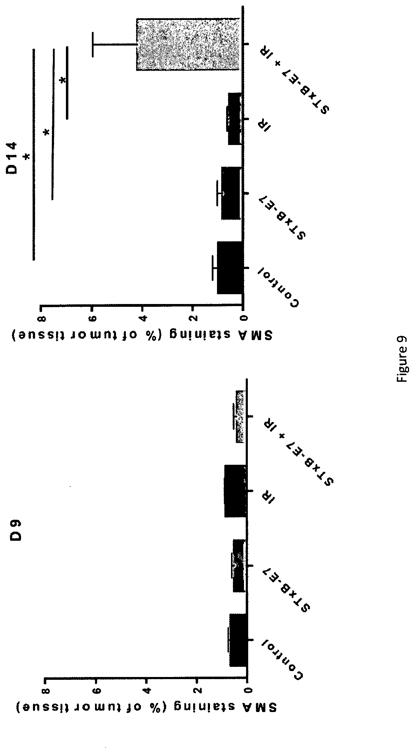

The figure 9 shows the mean ± SEM absorbance related to mean tumor perfusions depending on their treatment at day 9 (D9) or 14 (D14). *p<0.05 one-way ANOVA followed by Tukey's post-test.

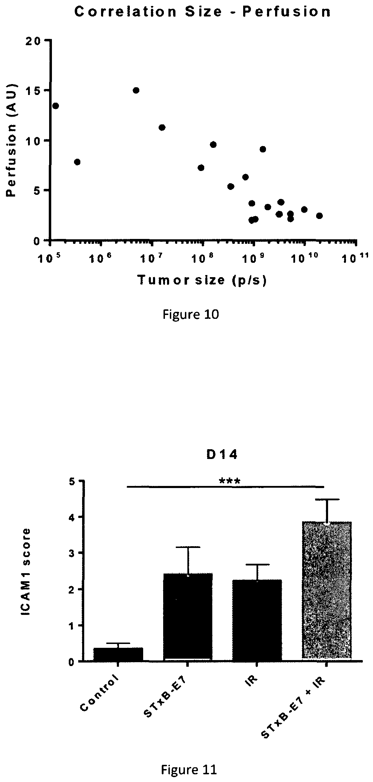

The figure 10 illustrates the data obtained by IVIS *in vivo* imaging at the day of sacrifice plotted against absorbance at day 14 for individual mice to show the correlation between tumor perfusion and size (p<0.001, Pearson correlation test).

These observations were supported by the Miles assay, a functional test that is performed to evaluate tumor perfusion. At 24 hours post irradiation (day 9), the perfusion of tumors was not yet affected by the treatments (Fig. 9, left panel); however, at day 14 after tumor challenge, tumor perfusion was significantly increased only by the combined treatment compared with both control and single therapies (Fig. 9, right panel). This observed increase was inversely correlated with the bioluminescent signal (p<0.05, Pearson's correlation test, Fig. 10), indicating that perfusion was linked to the tumor size at this time point.

Finally, vessel activation was evaluated by detecting ICAM-1 expression in the tumors by IHC. ICAM-1 is a transmembrane protein implied in the arrest and transmigration of leukocytes from blood vessels, which is upregulated in tumors from mice undergoing the different treatments.

The figure 11 shows the quantification of ICAM-1 expression based on a score from immunohistochemical staining. ***p<0.001 one-way ANOVA followed by Tukey's post-test.

The results show that ICAM1 is mainly upregulated in the combination group (Fig. 11).

Taken together, these data indicate that the combination of STxB-E7 immunization and IR promotes tumor vessel normalization and activation, thus favoring the recruitment and infiltration of leukocytes in the tumor tissue. Thus, for the first, a combination therapy based on local irradiation and vaccination induces increased tumor perfusion, which is accompanied by augmented pericyte coverage and ICAM-1 expression.

## Claims

1. A composition for use in the prevention or treatment of a subject suffering from a HPV-positive cancer in combination with radiotherapy, wherein said composition comprises a conjugate, eventually associated with pharmaceutically acceptable carrier, said conjugate comprising:
i) the Shiga Toxin B subunit (STxB), a fragment thereof having a length of at least 50 amino acids, which fragment binds to the glycolipid Gb3 receptor with a K_{D} equal or inferior to 10⁻⁷ M or a derivative thereof corresponding to an amino acid sequence binding to the glycolipid Gb3 receptor with a K_{D} equal or inferior to 10⁻⁷ M and having a percentage of identity of at least 95 % as compared to the amino acid SEQ id n°: 1; and
ii) at least one HPV epitope associated with said cancer.

2. The composition for use according to claim 1, wherein the HPV-positive cancer is a HPV-positive HNSCC.

3. The composition for use according to claim 1, wherein the HPV-positive cancer is a metastasis.

4. The composition for use according to any one of claim 1 to 3, wherein the Shiga Toxin B subunit (STxB) has the sequence SEQ id n°1, and a STxB fragment refers to the sequence SEQ id n°2.

5. The composition for use according to any claim 1 to 4, wherein said at least one HPV epitope is a HPV16 epitope.

6. The composition for use according to claim 5, wherein said at least one HPV16 epitope is from the E6 or E7 protein of HPV16, preferably said HPV16 epitope is from HPV16 E7 protein and is selected in the group comprising the peptides consisting of amino acids 5-18, 17-34, 35-50, 43-57, 43-77, 49-57, 50-62, 69-82 of the HPV 16 E7 protein.

7. The composition for use according to claim 6, wherein Preferably, said HPV16 epitope consists in the amino acids 43-57 of the HPV 16 E7 protein (SEQ id n°3; GQAEPDRAHYNIVTF).

8. The composition for use according to any claim 1 to 7, wherein i) the Shiga Toxin B subunit (STxB), fragment or derivative thereof; and ii) the at least one HPV cancer associated epitope are covalently linked using bifunctional protein coupling agents or in a fusion protein.

9. The composition for use according to claim 8, wherein i) the Shiga Toxin B subunit (STxB), fragment or derivative thereof; and ii) the at least one HPV cancer associated epitope are covalently linked using a bifunctional protein coupling agents.

10. The composition for use according to claim 9, wherein i) the Shiga Toxin B subunit (STxB) derivatives refer to the SEQ id n°4 and SEQ id n°5, preferably to SEQ id n°5.

11. The composition for use according to any claim 1 to 10, wherein i) the Shiga Toxin B subunit (STxB), fragment or derivative thereof; and ii) the at least one HPV cancer associated epitope are covalently linked in a fusion protein.

12. The composition for use according to any claim 1 to 11, wherein the radiotherapy can be applied at a dose in a range from about 10 to 55 Gy, preferably from about 15 to 50 Gy, such as 20 to 40Gy, concretely from about 20 to 35 Gy, and more concretely from about 25 to 30 Gy.

13. The composition for use according to any claim 1 to 12, wherein the composition and the radiotherapy are administrated currently, separately, or sequentially.

14. The composition for use according to any claim 1 to 13, wherein the composition is administrated before the irradiation.

15. The composition for use according to any claim 1 to 14, wherein the composition is for use in the prevention or treatment of a subject suffering from a cancer by a parenteral administration.

## Patentansprüche

1. Zubereitung zum Gebrauch in der Vorbeugung oder Behandlung eines Patienten, der unter einem HPV-positiven Krebs leidet, in Verbindung mit Strahlentherapie, wobei die genannte Zubereitung ein konjugiertes Protein umfasst, gegebenenfalls in Kombination mit einem pharmazeutisch verträglichen Träger, wobei das genannte konjugierte Protein umfasst:
i) die B-Untereinheit des Shiga-Toxin (STxB), ein Fragment davon, das eine Länge von mindestens 50 Aminosäuren aufweist, wobei dieses Fragment an den Glykolipidrezeptor Gb3 mit einer K_{D} gleich oder kleiner als 10⁻⁷ M bindet, oder ein Derivat davon, das einer Aminosäurensequenz entspricht, die an den Glykolipidrezeptor Gb3 mit einer K_{D} gleich oder kleiner als 10⁻⁷ M bindet und einen Identitätsanteil von mindestens 95% im Vergleich zur Aminosäurensequenz SEQ id Nr. 1 aufweist; und
ii) mindestens ein HPV-Epitop, das mit dem genannten Krebs verbunden ist.

2. Zubereitung zum Gebrauch nach Patentanspruch 1, wobei der HPV-positive Krebs ein HPV-positives HNSCC (Kopf-Hals-Plattenepithelkarzinom) ist.

3. Zubereitung zum Gebrauch nach Patentanspruch 1, wobei der HPV-positive Krebs eine Metastase ist.

4. Zubereitung zum Gebrauch nach irgendeinem der Patentansprüche 1 bis 3, in der die B-Untereinheit des Shiga-Toxin (STxB) der SEQ id Nr. 1 entspricht und ein STxB-Fragment sich auf die SEQ id Nr. 2 bezieht.

5. Zubereitung zum Gebrauch nach irgendeinem der Patentansprüche 1 bis 4, in der das genannte mindestens eine HPV-Epitop ein Epitop des HPV Typ 16 ist.

6. Zubereitung zum Gebrauch nach Patentanspruch 5, wobei das genannte mindestens eine Epitop des HPV Typ 16 aus dem Protein E6 oder E7 des HPV Typ 16 stammt, vorzugsweise stammt das genannte Epitop des HPV Typ 16 aus dem Protein E7 des HPV Typ 16 und wird aus der Gruppe gewählt, die die aus den Aminosäuren 5-18, 17-34, 35-50, 43-57, 43-77, 49-57, 50-62, 69-82 des Proteins E7 des HPV16 bestehenden Peptide umfasst.

7. Zubereitung zum Gebrauch nach Patentanspruch 6, in der das genannte Epitop des HPV Typ 16 vorzugsweise aus den Aminosäuren 43-57 des Proteins E7 des HPV16 (Seq id Nr. 3, GQAEPDRAHYNIVTF) besteht.

8. Zubereitung zum Gebrauch nach irgendeinem der Patentansprüche 1 bis 7, in der i) die B-Untereinheit des Shiga-Toxin (STxB), das Fragment oder Derivat davon, und ii) das mindestens eine, mit dem genannten HPV-Krebs verbundene Epitop, unter Verwendung bifunktionaler Proteinbindungsmittel oder in einem Fusionsprotein kovalent gebunden sind.

9. Zubereitung zum Gebrauch nach Patentanspruch 8, in der i) die B-Untereinheit des Shiga-Toxin (STxB), das Fragment oder Derivat davon, und ii) das mindestens eine, mit dem genannten HPV-Krebs verbundene Epitop, unter Verwendung eines bifunktionalen Proteinbindungsmittels kovalent gebunden sind.

10. Zubereitung zum Gebrauch nach Patentanspruch 9, in der sich Derivate der B-Untereinheit des Shiga-Toxin (STxB) auf die SEQ id Nr. 4 und SEQ id Nr. 5 beziehen, vorzugsweise auf SEQ id Nr. 5.

11. Zubereitung zum Gebrauch nach irgendeinem der Patentansprüche 1 bis 10, in der i) die B-Untereinheit des Shiga-Toxin (STxB), das Fragment oder Derivat davon, und ii) das mindestens eine, mit dem genannten HPV-Krebs verbundene Epitop, in einem Fusionsprotein kovalent gebunden sind.

12. Zubereitung zum Gebrauch nach irgendeinem der Patentansprüche 1 bis 11, in der die Strahlentherapie wird in einer Dosis zwischen etwa 10 und 55 Gy, vorzugsweise zwischen etwa 15 und 50 Gy, wie zwischen 20 und 40 Gy, speziell zwischen 20 und 35 Gy und insbesondere zwischen 25 und 30 Gy, angewendet.

13. Zubereitung zum Gebrauch nach irgendeinem der Patentansprüche 1 bis 12, in der die zubereitung und die Strahlentherapie werden gleichzeitig, getrennt oder nacheinander verabreicht.

14. Zubereitung zum Gebrauch nach irgendeinem der Patentansprüche 1 bis 13, in der die zubereitung wird vor der Strahlentherapie verabreicht.

15. Zubereitung zum Gebrauch nach irgendeinem der Patentansprüche 1 bis 14, in der die zubereitung wird zur Vorbeugung oder Behandlung eines an Krebs erkrankten Patienten durch parenterale Verabreichung verwendet.

## Revendications

1. Une composition pour une utilisation en combinaison avec une radiothérapie dans la prévention ou le traitement d'un sujet souffrant d'un cancer VPH-positif, laquelle composition comprend un conjugué, éventuellement associé à un support pharmaceutiquement acceptable, ledit conjugué comprenant :
i) La sous-unité B de la toxine Shiga (STxB), un fragment de celle-ci ayant une longueur d'au moins 50 acides aminés, lequel fragment se fixe au récepteur à glycolipide Gb3 avec un K_{D} égal ou inférieur à 10⁻⁷ M ou un dérivé de celui-ci correspondant à une séquence en acides aminés se fixant au récepteur à glycolipide Gb3 avec un K_{D} égal ou inférieur à 10⁻⁷ M et ayant un pourcentage d'identités d'au moins 95% en comparaison de la séquence d'acides aminés SEQ id n° :1 ; et
ii) Au moins un épitope de VPH associé audit cancer.

2. La composition pour une utilisation selon la revendication 1, dans laquelle le cancer VPH-positif est un HNSCC VPH-positif.

3. La composition pour une utilisation selon la revendication 1, dans laquelle le cancer VPH-positif est une métastase.

4. La composition pour une utilisation selon l'une quelconque des revendications 1 à 3, dans laquelle la sous-unité B de la toxine Shiga a la séquence SEQ id n°1, et le fragment STxB se réfère à la séquence SEQ id n°2.

5. La composition pour une utilisation selon l'une quelconque des revendications 1 à 4, dans laquelle l'épitope de HPV est un épitope d'HPV16.

6. La composition pour une utilisation selon la revendication 5, dans laquelle ledit au moins un épitope d'HPV16 est issu de la protéine E6 ou E7 d'HPV16, de préférence ledit épitope d'HPV16 est issu de la protéine E7 et est sélectionné dans le groupe comprenant les peptides consistant en les acides aminés 5-18, 17-34, 35-50, 43-57, 43-77, 49-57, 50-62, 69-82 de la protéine E7 d'HPV16.

7. La composition pour une utilisation selon la revendication 6, dans laquelle de préférence ledit épitope HPV16 consiste en la séquence d'acides aminés 43-57 de la protéine E7 d'HPV16 (SEQ id n°3 ; GQAEPDRAHYNIVTF).

8. La composition pour une utilisation selon l'une quelconque des revendications 1 à 7, dans laquelle i) la sous-unité B de la toxine Shiga (STxB), le fragment ou le dérivé de celle-ci ; et ii) le au moins un épitope de VPH associé au cancer sont liés covalemment en utilisant des agents de couplage de protéines bifonctionnels ou une protéine de fusion.

9. La composition pour une utilisation selon la revendication 8, dans laquelle i) la sous-unité B de la toxine Shiga (STxB), le fragment ou le dérivé de celle-ci ; et ii) le au moins un épitope de VPH associé au cancer sont liés covalemment en utilisant des agents de couplage de protéines bifonctionnels.

10. La composition pour une utilisation selon la revendication 9, dans laquelle i) les dérivés de la sous-unité B de la toxine Shiga (STxB) se réfèrent à SEQ id n°4 et SEQ id n°5, de préférence SEQ id n°5.

11. La composition pour une utilisation selon l'une quelconque des revendications 1 à 10, dans laquelle i) la sous-unité B de la toxine Shiga (STxB), le fragment ou le dérivé de celle-ci ; et ii) le au moins un épitope de VPH associé au cancer sont liés covalemment en utilisant une protéine de fusion.

12. La composition pour une utilisation selon l'une quelconque des revendications 1 à 11, dans laquelle la radiothérapie est appliquée à une dose comprise entre environ 10 et 55 Gy, de préférence entre environ 15 et 50 Gy, comme entre 20 et 40 Gy, concrètement entre 20 et 35 Gy et, plus concrètement entre 25 et 30Gy.

13. La composition pour une utilisation selon l'une quelconque des revendications 1 à 12, dans laquelle la composition et la radiothérapie sont administrées simultanément, séparément ou consécutivement.

14. La composition pour une utilisation selon l'une quelconque des revendications 1 à 13, dans laquelle la composition est administrée avant l'irradiation.

15. La composition pour une utilisation selon l'une quelconque des revendications 1 à 14, dans laquelle la composition est utilisée pour une utilisation dans la prévention ou le traitement d'un sujet souffrant d'un cancer par une administration parentérale.
